# EUROPEAN PATENT SPECIFICATION

(11) **EP 1 573 049 B1**
(45) Date of publication and mention of the grant of the patent: **29.04.2009**
(21) Application number: 03782609.6
(22) Date of filing: 09.12.2003
(51) Int. Cl.: C12Q 1/18, C12Q 1/48, C12N 5/10

(54) **MSS4 AS AN ANTIFUNGAL TARGET**
MSS4 ALS ANTIFUNGISCHES ZIELMOLEKÜL
UTILISATION DE LA MSS4 COMME CIBLE ANTIFONGIQUE

(30) Priority: 09.12.2002 GB 0228706
(43) Date of publication of application: 14.09.2005
(73) Proprietor: UCB, S.A., 1070 Bruxelles (BE)
(72) Inventor: HAYDON, David, John, Oxfordshire OX14 4RY (GB)
(74) Representative: Thompson, John
(86) International application number: PCT/GB2003/005376
(87) International publication number: WO 2004/053150

(56) References cited:
- CA-A- 2 309 327
- HOMMA KEIICHI ET AL: "Phosphatidylinositol-4-phosphate 5-kinase localized on the plasma membrane is essential for yeast cell morphogenesis" JOURNAL OF BIOLOGICAL CHEMISTRY, vol. 273, no. 25, 19 June 1998 (1998-06-19), pages 15779-15786, XP002274409 ISSN: 0021-9258
- DESRIVIERES SYLVANE ET AL: "MSS4, a phosphatidylinositol-4-phosphate 5-kinase required for organization of the actin cytoskeleton in Saccharomyces cerevisiae" JOURNAL OF BIOLOGICAL CHEMISTRY, vol. 273, no. 25, 19 June 1998 (1998-06-19), pages 15787-15793, XP002274410 ISSN: 0021-9258
- HAIRFIELD MICHELLE L ET AL: "Phosphatidylinositol-4-phosphate 5-kinase activity is stimulated during temperature-induced morphogenesis in Candida albicans" MICROBIOLOGY (READING), vol. 148, no. 6, June 2002 (2002-06), pages 1737-1746, XP002274411 ISSN: 1350-0872
- ENLOE BRIAN ET AL: "A single-transformation gene function test in diploid Candida albicans" JOURNAL OF BACTERIOLOGY, vol. 182, no. 20, October 2000 (2000-10), pages 5730-5736, XP002274412 ISSN: 0021-9193 cited in the application

## Description

The present invention relates to a novel antifungal target, 1-phosphotidylinositol-4-phosphate 5-kinase (MSS4), of use in screening methods for MSS4 inhibitors. The use thereof as antifungal compounds, pharmaceutical compositions containing them and their use in medicine, specifically in the treatment of an individual susceptible to or suffering from an anti-fungal infection, is also described. In particular the compounds find use in the treatment of topical or mucosal (e.g. thrush and vaginal candidiasis) fungal infections, e.g. caused by fungus of the *Candida* species, and for systemic infections, e.g. caused by fungi of *Candida* species, such as *C*. *albicans.*

### INTRODUCTION

### Fungal Pathogens

Major fungal pathogens include those of the *Candida* species, such as *C. albicans*.

Fungal infections can affect humans and animals. Generally, fungal infections occur as a result of opportunistic infection of a weakened or immune-suppressed individual and these can include infections of the joints and skin. The yeast *Candida albicans* (*C. albicans*) is one of the most pervasive fungal pathogens in humans. It is the cause of an increasing financial and logistic burden on the medical care system and its providers. Although *C. albicans* is a member of the normal flora of the mucous membranes in the respiratory, gastrointestinal, and female genital tracts, it may gain dominance in such locations (e.g. upon treatment with antibacterial antibiotics, in patients with diabetes or in patients using corticosteroids) and be associated with pathologic conditions. In addition, almost all HIV-positive individuals suffer from a *Candida* infection prior to the onset of developing full-blown AIDS. The incidence of life-threatening fungal infections has increased dramatically as the population of immunocompromised individuals (including cancer, organ transplant and AIDS patients) has increased. Present therapeutic options for the treatment of these infections are limited and thus there is a need for new anti-fungal compounds with novel mechanisms of action for use in treating or preventing such fungal infections.

Antifungal drug development often relies on the screening of a large number of compounds before one or more lead compounds are found that are effective against the target fungi. Thus, it is critical for the development of these screens to define proteins essential for survival or growth of the target fungi and to discover means of purifying or producing such proteins. Thus, there is a need in the art to identify essential fungal structural or functional gene products that can serve as targets for drug intervention, and for methods for identifying useful anti-fungal agents that impair the function of these essential fungal gene products, and for compositions that can be used to treat fungal infections by preventing or inhibiting the growth of, and preferentially killing, the fungi.

### Identification of 'Essential'genes

Varying definitions are used in the art for what constitutes an essential gene, but the term is most frequently applied to those genes necessary for growth on rich medium. This variation in the art can be misleading and restrictive in terms of identifying gene products that constitute good antifungal targets. A significant amount of *C. albicans* genomic sequence information is available in both public (http://www.sequence.stanford.edu/group/candida/) and private (Incyte Genomics Inc.) databases. This can be combined with genomic sequence data from other organisms (The yeast genome directory, 1997, Nature, 387(6632 Suppl):5; Wood V, et al, 2002, Nature, 415(6874):871-80) and with supporting data such as the functional profiling of the *Saccharomyces cerevisiae* genome (Giaever G, et al., 2002, Nature, 418(6896):387-91). This bioinformatics driven approach has allowed the prediction of genes that may be essential in *C. albicans* (Spaltmann F, et al., 1999, Drug Discovery Today, 4:17-26). However, even for relatively closely related organisms such as *Saccharomyces cerevisiae* and *C. albicans*, there are significant differences that make such *in silico* predictions unreliable. For example, CET1 and CDC25 are not essential in *C. albicans* despite being essential in *Saccharomyces cerevisiae* (Enloe B, et al., 2000, J Bacteriol, Oct, 182:20, 5730-6; Dunyak DS, et al., 2002, 6th ASM Conference on Candida and Candidiasis).

There are several strategies for identifying essential genes in C. *albicans* by practical methodology. Negative approaches rely on the inability to generate a strain that contains a disrupted functional target gene. The majority of genes characterised in this way rely on variations of the *URA* blaster method (Fonzi, W.A. & Irwin, MY, 1993, Genetics, 134:717-728). These techniques can be highly effective for analysing individual genes, but they may not be completely reliable. *CET1* was incorrectly reported to be essential in *C. albicans* because viable homozygous mutants could not be recovered using the *URA* blaster method (Pei, *et al*, 2001). However it has subsequently been shown not to be essential (Dunyak, *et al*, 2002). Positive approaches control the expression of the target gene either indirectly, such as using antisense RNA (De Backer MD, et al., 2001, Nat Biotechnol, Mar, 19:3, 235-41), or directly such as promoter replacement with inducible promoters such as *MRP1* and *Tet* (Munro CA, et al., 2001, Mol Microbiol, Mar 39:5 1414-26; Nakayama H, et al., 2000, Infect Immun, Dec 68:12 6712-9).

Genome wide identification of essential genes has not been successfully applied to *C. albicans* for several reasons. These include that *C. albicans* is a diploid organism, is not capable of mating under normal circumstances, and that there are few functional transposable elements. Attempts to overcome these issues by using antisense RNA and promoter interference have had limited success (De Backer, *et al*, 2001). Therefore there is a need in the art for validated essential genes of fungal species, in particular the Candida species, that can be used as targets for the development of new antifungal compounds.

### 1-Phosphotidylinositol-4-phosphate 5-kinase

1-Phosphotidylinositol-4-phosphate 5-kinase (MSS4) E.C. 2.7.1.68 is involved in inositol phosphate metabolism and functions to convert ATP + 1-phosphotidyl-1D-myo-inositol 4-phosphate → ADP + 1-phosphotidyl-1D-myo-inositol 4,5-bisphosphate (PIP2) (Yoshida et al., 1994, Mol. Gen. Genet. 242, 631-640; Hairfield et al., 2002, Microbiology, 148(6), 1737-1746). The MSS4 enzyme is encoded by the MSS4 gene (YDR208W/ YD8142A.05) and details for the fungal enzyme are provided under Accession numbers: P38994 in the Swiss Prot database (http://ca.expasy.org) for *S. cerevisiase*; CA0623 in the Institut Pasteur *Candida* database (http://genolist.pasteur.fr/CandidaDB/) which is cross-referenced with the Stanford open-reading frame (ORF) orf6.1660 (http://www.sequence.stanford.edu/group/candida/). Synonyms for MSS4 include diphosphoinositide kinase, PIP kinase (PIP5K), PtdIns(4)P-5-kinase and CaMSS4.3.

Homma K, et al, 1998, J.Biol. Chem., 273:25, 15779-15786 and Desrivieres S, et al, 1998, J. Biol. Chem. 273:25, 15787-15793 describe the role of MSS4 in actin organisation in *S*. *cerevisiase*.

CA-A-2309327 relates to the finding that the protein PKA-R is essential to the survival of *C. albicans* and accordingly that PKA-R constitutes a cellular target for the development of anti-*Candida* substances.

The present invention is based on the finding that MSS4 is an essential protein for the fungal species *Candida*. This finding demonstrates the potential for developing fungal selective MSS4 inhibitors, which can kill invading fungal organisms while sparing the host of any detrimental effects. Prior to this invention, MSS4 has not been considered as a differential target for antifungal compounds.

### SUMMARY OF THE INVENTION

The present invention relates to the use of fungal 1-phosphotidylinositol-4-phosphate 5-kinase (hereinafter referred to as "MSS4") as a target for antifungal therapy, in particular, for antifungal therapy against *Candida* species, in a method for screening or testing for potential antifungal compounds, e.g. small molecules, by determining whether a candidate agent is capable of specifically inhibiting fungal phosphorylation activity via a selective interaction with MSS4. Described herein is the essential nature of MSS4 in *C. albicans*. Further described herein is the use of mechanism-based assays, with or without the use of a transformed eukaryotic organism with the MSS4 gene under the control of a heterologous promoter, to facilitate drug discovery.

Additionally, described herein are MSS4 inhibitor compositions and methods for treating fungal infections, e.g. *Candida* fungal infections, by administering to a host suffering from a fungal infection a therapeutically effective amount of a MSS4 inhibitor.

### Definitions

In the context of this invention:
"Essential gene" is defined as a fungal gene necessary for growth on rich medium.
"MSS4 inhibitor" is defined as any compound that impairs MSS4 function in the fungus. A compound that impairs MSS4 function may be one that, modulates, e.g. inhibits, the expression or activity of MSS4, interacts with MSS4 or binds to MSS4. Furthermore, a compound that modulates the expression of MSS4 may interfere with the transcription of the gene encoding MSS4 or with the translation of mRNA encoding MSS4 in target organisms. It is desirable that the compound shows specificity for fungal over host MSS4. A therapeutically effective amount of a MSS4 inhibitor is one that is sufficient to inhibit partially or fully the phosphorylation activity via MSS4 of the causative fungi.
"Fragment" is defined as a fragment of a MSS4 polypeptide e.g. as provided by accession numbers P38994, CA0623, or Stanford orf6.1660, having at least 70%, more preferably it has at least 75%, at least 80%, at least 85%, at least 90%, at least 95% or at least 98% identity to the native polypeptide over the length of the fragment and which is at least ten amino acids long. An active fragment is one that retains the ability to carry out the MSS4 enzyme function.
"Function-conservative fragment" is defined as a MSS4 encoding sequence in which a given amino acid residue in the polypeptide has been changed without altering the overall conformation and function of the native polypeptide, including, but not limited to, replacement of an amino acid with one having similar physical and/or chemical properties (such as, for example, acidic, basic, hydrophobic, and the like) or polymorphisms.
"Fusion protein" unless otherwise specified, is defined as a MSS4 polypeptide, fragment or function-conservative fragment thereof fused via a covalent bond (e.g. a peptide bond), at optionally the N-terminus or the C-terminus, to an amino acid sequence of another protein (or portion thereof; preferably at least a 10, 20 or 50 amino acid portion of the protein). Preferably the polypeptide, or fragment thereof, is linked to the other protein at the N-terminus of the constant domain of the polypeptide.
"Growth" is defined as the normal growth pattern of fungi, i.e. the cell doubling time during the log phase of growth. For example, in rich media, wild-type *C. albicans* has a doubling time of approximately 60 minutes. Growth of the cells may be measured by following the optical density of cells in liquid media, where an increasing optical density indicates growth. Alternatively, growth can also be measured by colony formation from single cells on solid media plates.
"Viability" is defined as the ability of fungal cells to resume growth following a treatment of the cells that results in cessation of growth. One typical means by which viability is measured is by testing the ability of cells to form colonies on solid media plates.

### DETAILED DESCRIPTION OF THE INVENTION

The invention provides *C. albicans* MSS4 as a specific target for antifungal compounds.

The methods of the invention provide a facile and specific assay to screen compounds as potential antifungal compounds against *Candida* species.

Thus, the invention provides a method of screening or testing for candidate compounds against *Candida* species, that impair *C. albicans* MSS4 1-phosphotidylinositol-4-phosphate 5-kinase enzyme function (MSS4), comprising:
a) providing *C*. *albicans* MSS4;
b) providing one or more candidate compounds;
c) contacting said MSS4 with said one or more candidate compounds; and
d) determining the interaction of the candidate compound with said MSS4.

The screening method of the invention may be performed using techniques known in the art, e.g. the assay may comprise a growth inhibition assay, a binding assay or a translation inhibition assay. Binding assays include competitive binding assays, wherein the binding affinity of the candidate compound is compared with that of a known enzyme substrate for MSS4, a preferred enzyme substrate is adenosine triphosphate (ATP).

In the screening methods of the invention the candidate compound or enzyme substrate may be labelled to allow easy quantitation of the interaction between the candidate compound and the enzyme. Preferably the substrate is labelled e.g. using a radiolabel, such as but not limited to, ³³P and the preferred substrate is adenosine triphosphate (ATP), as described in Example 3.

MSS4 may be cloned or purified from fungi for use in *in vitro* binding, ligand binding or translation inhibition assays. The MSS4 is from *C. albicans*, a fungal pathogen of humans and animals. In a particular embodiment, MSS4 may comprise a fragment, a function-conservative variant, an active fragment or a fusion protein of MSS4.

MSS4 can be purified by techniques well known to those skilled in the art. Methods for polypeptide purification include, without limitation, preparative disc-gel electrophoresis, isoelectric focusing, HPLC, reversed-phase HPLC, gel filtration, ion exchange and partition chromatography, and countercurrent distribution. For some purposes, it is preferable to produce the polypeptide in a recombinant system in which the protein contains an additional sequence tag that facilitates purification, such as, but not limited to, a polyhistidine sequence. The polypeptide can then be purified from a crude lysate of the host cell by chromatography on an appropriate solid-phase matrix. Alternatively, antibodies produced against the fungal target protein or against peptides derived therefrom can be used as purification reagents.

MSS4 can also be provided in a transformed eukaryotic organism under the control of a heterologous promoter. Such cells can be used in growth inhibition assays. The eukaryotic organism is *C.albicans*. Briefly, a *C.albicans* strain is generated in which expression of the MSS4 gene can be tightly regulated. To do this the wild-type allele of the gene of interest is replaced with an allele that can be regulated by an exogenous agent. In general, nucleic acid manipulations and other related techniques used in practicing the present invention employ methods that are well known in the art, as disclosed in, e.g. Molecular Cloning, A Laboratory Manual (2nd Ed., Sambrook, Fritsch and Maniatis, Cold Spring Harbor) and Current Protocols in Molecular Biology (Eds.Ausubel, Brent, Kingston, More, Feidman, Smith and Stuhl, Greene Publ. Assoc., Wiley Interscience, NY, NY, 1997).

Thus, the invention also provides a modified eukaryotic *C. albicans* cell(s) wherein the cell(s) expresses *C. albicans* MSS4 under the control of a heterologous promoter. In one embodiment, the MSS4 may be heterologous or homologous. Preferably, the MSS4 is homologous.

The eukaryotic cell is *C.albicans*.

In a specific embodiment, MSS4 may be expressed in a tetracycline-regulatable expression system (as described in Example 4). The tetracycline-regulatable expression system is an established tool for conditional expression of eukaryotic genes (Gossen, MA, & H Bujard, 1992, Proc. Natl. Acad. Sci. USA, 89:5547-5551; Nagahashi S, et al., 1997, Mol. Gen. Genet, 255:372-375; Nakayama H, et a., 1998, Microbiology, 144:2407-2415; Nakayama H, et al., 2000, Infect Immun, Dec 68:12 6712-9). Such a system consists of two components derived from the Tn10 transposon of *Escherichia coli* (Hillen W, & A Wissmann, 1989, In Protein-nucleic acid interaction, vol. 10, Macmillan Press, London, United Kingdom, p. 143-162). The first component comprises a minimal promoter element downstream of a tetracycline operator sequence (*tetO*), which replaces the natural promoter of the target gene. The second component is a transactivator, that is a fusion protein comprising a transcriptional activation domain and the tetracycline repressor protein (*TetR*).

In the absence of tetracycline, *TetR* specifically binds to *tetO* as a dimer, resulting in the activation of transcription by recruiting the transactivator to the promoter. When tetracycline is present, it binds to the TetR repressor with high affinity and inhibits dimerisation, thereby preventing binding to *tetO*. Therefore, MSS4 gene expression is activated in the absence, and repressed in the presence, of tetracycline. A synthetic tetracycline derivative, such as but not limited to, doxycycline can be used to control the expression of the MSS4 gene as described above.

The tetracycline-regulatable expression system has several advantages over alternative systems. It was derived from a prokaryotic system so it is not anticipated to show pleiotropic effects (Gossen and Bujard, 1992), it can be used in an animal host (Nakayama H, et al., 1998, Microbiology, 144:24072415; Nakayama H, et al., 2000, Infect. Immun., Dec 68:12 6712-9), it is highly specific, and non-toxic.

Such modified cells may be used in screening methods. Thus, the invention also provides a method of screening or testing for candidate compounds against *Candida* species, that impair *C*. *albicans* MSS4 1-phosphotidylinositol-4-phosphate 5-kinase enzyme (MSS4) function, comprising;
a) providing *C. albicans* MSS4 in a eukaryotic *C*. *albicans* cell(s) that expresses *C. albicans* MSS4 under the control of a heterologous promoter;
b) providing one or more candidate compounds;
c) contacting said eukaryotic cell(s) with said one or more candidate compounds; and
d) determining the interaction of the candidate compound with said MSS4 by assessing the effect on growth or viability of said cells.

The screening methods of the invention include both *in vitro* and *in vivo* methods. Candidate compounds which may be screened according to the methods of the invention include small molecules and peptides. The candidate compounds may be synthetic compounds, a mixture of synthetic compounds, a crude preparation, a purified preparation or an initial extract of a natural product obtained from plant, microorganism or animal sources.

A compound identified by the screening methods described above, which impairs *C. albicans* MSS4 function, is referred to herein as a 'MSS4 inhibitor'.

MSS4 inhibitors identified by the screening methods of the invention are useful as antifungal compounds. Thus, they may be used in the treatment and prevention of various fungal infections such as topical or mucosal (e.g. thrush and vaginal candidiasis) fungal infections, caused by *Candida* species, and for systemic fungal infections, caused by *Candida* species, such as *C*. *albicans.*

The medicament identified by the screening methods of this invention can be used in the curative or prophylatic treatment of fungal infections in humans and animals, especially domestic animals such as dogs, cats, horses etc.

In addition, the MSS4 inhibitors identified by the screening methods of this invention also find use in the curative or prophylatic treatment of fungal infections in subjects who are immunosuppressed e.g. as a result of a therapy (e.g. chemotherapy or radiotherapy), organ transplant or an infection (e.g. HIV).

In order to use MSS4 inhibitors in therapy (human or veterinary), they will normally be formulated into a pharmaceutical composition in accordance with standard pharmaceutical practice, e.g. by admixing the MSS4 inhibitor and a pharmaceutically acceptable carrier.

The pharmaceutical compositions are particularly useful in the prevention or treatment of *Candida* fungal infections.

MSS4 inhibitors may be administered to a host by any of the routes conventionally used for drug administration, for example they may be administered parenterally, orally, topically (including buccal, sublingual or transdermal) or by inhalation. The most suitable route for administration in any given case will depend on the particular MSS4 inhibitor, the infectious organism involved, the host, and the nature and severity of the disease and the physical condition of the host.

The MSS4 inhibitors may be administered in combination, e.g. simultaneously, sequentially or separately, with one or more other therapeutically active, e.g. antifungal, compounds.

The dosage to be administered of a MSS4 inhibitor will vary according to the particular MSS4 inhibitor, the infectious organism involved, the host, the severity of the disease, physical condition of the host, and the selected route of administration; the appropriate dosage can be readily determined by a person skilled in the art. For the treatment of fungal diseases in humans and animals, the dosage may range from 0.01 mg/kg to 750 mg/kg. For prophylactic use in human and animals, the dosage may range from 0.01 mg/kg to 100 mg/kg. ,

The compositions may contain from 0.1 % by weight, preferably from 10-60% by weight, of the MSS4 inhibitor, depending on the method of administration.

Pharmaceutical compositions may be conveniently presented in unit dose forms containing a predetermined amount of MSS4 inhibitor per dose. Such a unit may contain for example but without limitation, 100mg/kg to 0.1mg/kg depending on the condition being treated, the route of administration and the age, weight and condition of the host. Preferred unit dosage compositions are those containing a daily dose or sub-dose, as recited above, or an appropriate fraction thereof, of the active ingredient.

It will be recognized by one of skill in the art that the optimal quantity and spacing of individual dosages will be determined by the nature and extent of the condition being treated, the form, route and site of administration, and the particular host being treated, and that such optimums can be determined by conventional techniques. It will also be appreciated by one of skill in the art that the optimal course of treatment, i.e. the number of doses given per day for a defined number of days, can be ascertained by those skilled in the art using conventional course of treatment determination tests.

Dosage regimens are adjusted to provide the optimum desired response. For example, a single bolus may be administered, several divided doses may be administered over time or the dose may be proportionally reduced or increased as indicated by the exigencies of the therapeutic situation.

Pharmaceutically acceptable carriers may take a wide variety of forms depending, e.g. on the route of administration.

Compositions for oral administration may be liquid or solid. Oral liquid preparations may be in the form of, for example, aqueous or oily suspensions, solutions, emulsions, syrups or elixirs, or may be presented as a dry product for reconstitution with water or other suitable vehicle before use. Oral liquid preparations may contain suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; water; non-aqueous vehicles (which may include edible oils), for example almond oil, oily esters such as glycerine, propylene glycol, or ethyl alcohol; preservatives, for example methyl or propyl p-hydroxybenzoate or sorbic acid; flavoring agents, preservatives, coloring agents and the like may be used.

In the case of oral solid preparations such as powders, capsules and tablets, carriers such as starches, sugars, microcrystalline cellulose, diluents, granulating agents, lubricants, binders, disintegrating agents, and the like may be included. Because of their ease of administration, tablets and capsules represent the most advantageous oral dosage unit form in which case solid pharmaceutical carriers are generally employed. In addition to the common dosage forms set out above, MSS4 inhibitors may also be administered by controlled release means and/or delivery devices. Tablets and capsules may comprise conventional carriers or excipients such as binding agents for example, syrup, acacia, gelatin, sorbitol, tragacanth, or polyvinylpyrrolidone; fillers, for example lactose, sugar, maize-starch, calcium phosphate, sorbitol or glycine; tableting lubricants, for example magnesium stearate, talc, polyethylene glycol or silica; disintegrants, for example potato starch; or acceptable wetting agents such as sodium lauryl sulphate. The tablets may be coated by standard aqueous or non-aqueous techniques according to methods well known in normal pharmaceutical practice.

Pharmaceutical compositions suitable for oral administration may be presented as discrete units such as capsules, cachets or tablets, each containing a predetermined amount of the active ingredient, as a powder or granules, or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier, which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients.

Compressed tablets may be prepared by compressing, in a suitable machine, the active ingredient in a free-flowing form such as a powder or granules, optionally mixed with a binder, lubricant, inert diluent, surface active or dispersing agent. Moulded tablets may be made by moulding, in a suitable machine, a mixture of the powdered compound moistened with an inert liquid diluent. Desirably, each tablet contains from about 1 mg to about 500 mg of the active ingredient and each cachet or capsule contains from about 1 to about 500 mg of the active ingredient.

Compositions comprising a MSS4 inhibitor may also be prepared in powder or liquid concentrate form. Conventional water soluble excipients, such as lactose or sucrose, may be incorporated in the powders to improve their physical properties. Thus, particularly suitable powders comprise 50 to 100% w/w, and preferably 60 to 80% w/w of the combination and 0 to 50% w/w and preferably 20 to 40% w/w of conventional excipients. When used in a veterinary setting such powders may be added to animal feedstuffs, for example by way of an intermediate premix, or diluted in animal drinking water.

Liquid concentrates for oral administration suitably contain a water-soluble compound combination and may optionally include a pharmaceutically acceptable water miscible solvent, for example polyethylene glycol, propylene glycol, glycerol, glycerol formal or such a solvent mixed with up to 30% v/v of ethanol.

Pharmaceutical compositions suitable for parenteral administration may be prepared as solutions or suspensions of the MSS4 inhibitors in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include aqueous or non-aqueous sterile injection solutions which may contain anti-oxidants, buffers, bacteriostats and solutes which render the composition isotonic with the blood of the intended recipient, and aqueous and non-aqueous sterile suspensions which may include suspending agents and thickening agents. Extemporaneous injection solutions, dispersions and suspensions may be prepared from sterile powders, granules and tablets.

The compositions may be presented in unit-dose or multi-dose containers, for example in sealed ampoules and vials and to enhance stability, may be stored in a freeze-dried (lyophilized) condition requiring only the addition of the sterile liquid carrier, for example water for injections, immediately prior to use. The sterile liquid carrier may be supplied in a separate vial or ampoule and can be a solvent or dispersion medium containing, for example, water, ethanol, polyol (e.g. glycerol, propylene glycol and liquid polyethylene glycol), suitable mixtures thereof, and vegetable oils. Advantageously, agents such as a local anaesthetic, preservative and buffering agents can be included the sterile liquid carrier.

Pharmaceutical compositions adapted for topical administration may be formulated as ointments, creams, suspensions, lotions, powders, solutions, pastes, gels, impregnated dressings, sprays, aerosols or oils, transdermal devices, dusting powders, and the like. These compositions may be prepared via conventional methods containing the active ingredient. Thus, they may also comprise compatible conventional carriers and additives, such as preservatives, solvents to assist drug penetration, emollients in creams or ointments and ethanol or oleyl alcohol for lotions. Such carriers may be present as from about 1% up to about 98% of the composition. More usually they will form up to about 80% of the composition. As an illustration only, a cream or ointment is prepared by mixing sufficient quantities of hydrophilic material and water, containing from about 5-10% by weight of the compound, in sufficient quantities to produce a cream or ointment having the desired consistency.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis.

For applications to external tissues, for example the mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical compositions adapted for topical administration in the mouth include lozenges, pastilles and mouth washes.

Pharmaceutical compositions adapted for topical administration to the eye include eye drops wherein the active ingredient is dissolved or suspended in a suitable carrier, especially an aqueous solvent. They also include topical ointments or creams as above.

Pharmaceutical compositions suitable for rectal administration wherein the carrier is a solid are most preferably presented as unit dose suppositories. Suitable carriers include cocoa butter or other glyceride or materials commonly used in the art, and the suppositories may be conveniently formed by admixture of the combination with the softened or melted carrier(s) followed by chilling and shaping moulds. They may also be administered as enemas.

Pharmaceutical compositions adapted for vaginal administration may be presented as pessaries, tampons, creams, gels, pastes, foams or spray compositions. These may comprise emollients or bases as commonly used in the art.

The following examples are to be construed as merely illustrative and not a limitation on the scope of the invention in any way. The specification refers to the figure in which:

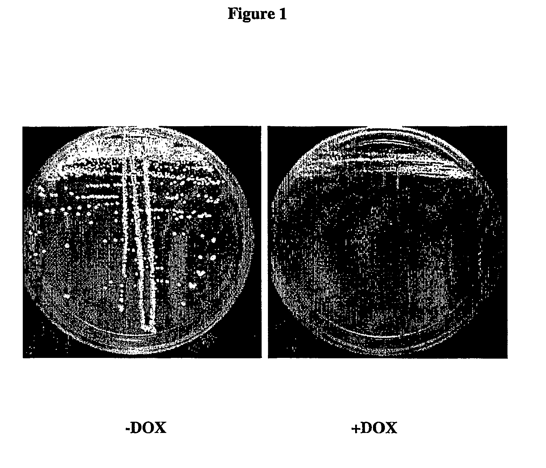

**Figure 1**: This shows the solid phase growth of a recombinant *C.albicans* strain wherein the MSS4 gene is under the regulation of a tetracycline-repressible promoter. The images represent two days growth at 30°C on SC-U plates with, either no supplement (-DOX), or supplemented with the tetracycline analogue doxycycline 20µg/ml (+DOX). The effect of strong induction or tight repression of MSS4 gene expression on colony formation is observed in the absence and presence of doxycycline, respectively.

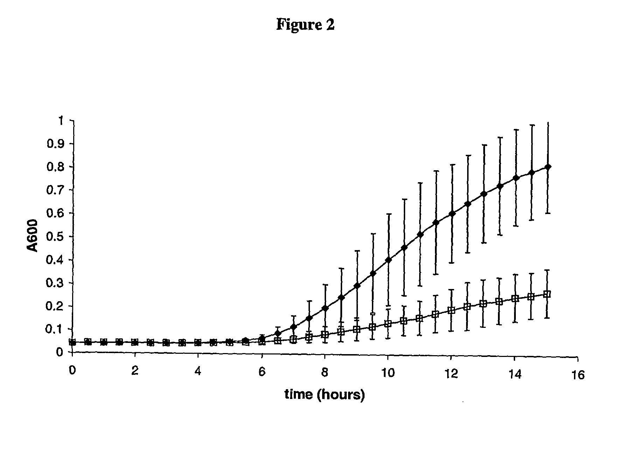

**Figure 2****:** This shows the liquid phase growth of a recombinant *C.albicans* strain wherein the MSS4 gene is under the regulation of a tetracycline-repressible promoter. Growth is in YEPD medium at 25°C with, either no supplement (-DOX, ◆ ± S.D), or supplemented with the tetracycline analogue doxycycline, 20µg/ml (+DOX, □ ± S.D.). The effect of strong induction or tight repression of MSS4 gene expression on growth is observed in the absence and presence of doxycycline, respectively.

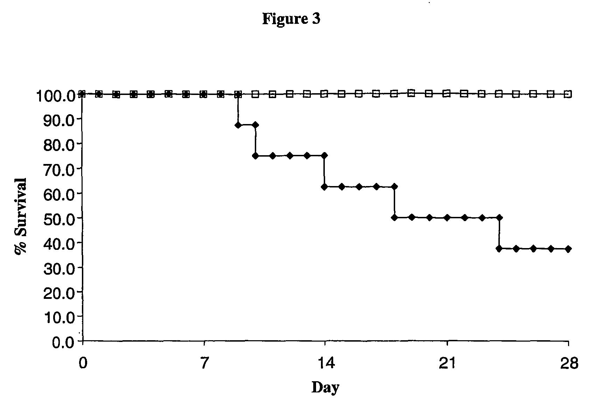

**Figure 3**: This shows the survival rate of mice infected with the recombinant *C.albicans* strain wherein the MSS4 gene is under the regulation of a tetracycline-repressible promoter. Where indicated, mice were administered with either water (control ◆) or with 2mg/ml of the tetracycline analogue doxycyline dissolved in 5% sucrose solution (+DOX, □), as drinking water. Under the conditions where the MSS4 gene was expressed (control) the C. *albicans* was able to affect the survival rate of the mice, whereas under the conditions where MSS4 expression was repressed (i.e. in the presence of doxycycline) the C. *albicans* was unable to impact on the survival rate of the mice.

### EXAMPLES

### Example 1: Expression of MSS4

The MSS4 ORF was cloned into pGex-6P-1 (Pharmacia Biotech) to enable expression as a 5' GST fusion protein. Host *E.coli* used were Tuner(DE3)pLysS (Novagen). *E.coli* harbouring the expression plasmid were grown at 37°C to mid log phase (OD600 = 0.6) then cooled to 17°C and induced with IPTG (0.3mM) for approximately 4 h.

### Example 2: Purification of MSS4

*E. coli* cells from 5L of culture were harvested by centrifugation at 6000 x g for 10min. The cell pellets were frozen on -80°C, thawed and resuspended in 150ml of buffer A (20mM Hepes (pH7.4), 5mM DTT, 140mM NaCl, 1mM EDTA, 10% (v/v) glycerol, 0.02% (w/v) sodium azide, and a protease inhibitor cocktail consisting of 1mM benzamidine, 1mg.ml⁻¹ each of pepstatin, antipain and leupeptin, 0.2mM PMSF, Complete (Roche) and general protease inhibitor cocktail (Sigma).

The extract was sonicated in 3x10s bursts to reduce viscosity. Triton X-100 was added to 1% followed by centrifugation at 75 000 x g for 10min. The supernatant was batch loaded onto 5ml glutathione Sepharose (Amersham Biosciences) at 4°C. The resin was extensively batch washed with phosphate buffer containing 0.5M NaCl then with buffer B (20mM Hepes (pH 7.4), 1mM DTT, 1mM EDTA, 100mM NaCl, 10% glycerol, 0.02% sodium azide). The resin was then packed into a disposable PD-10 column. MSS4 was cleaved from GST by incubation of the glutathione Sepharose beads with 1 column volume of buffer B containing 300U Precision protease (Amersham Biosciences) overnight at 4°C with constant tumbling. MSS4 was recovered by centrifugation of the PD-10 column (500 x g) followed by washing of the column with buffer B.

Fractions containing MSS4 were pooled and concentrated to 2ml using a Vivaspin 6 with a 10K cut-off membrane (Vivascience). The protein was applied to a Superdex 200 26/10 column linked to an AKTA FPLC system (Amersham Biosciences) and 1ml fractions were collected. Eluted fractions containing MSS4 were pooled, concentrated and diluted 10-fold with buffer without NaCl and applied to a Mono Q column (HR5/5) linked to an AKTA FPLC system (Amersham Biosciences). MSS4 was eluted using a linear 0-500mM NaCl gradient and MSS4 was eluted at approximately 250-350 mM NaCl.

### Example 3: MSS4 assay

The 1-phosphotidylinositol-4-phosphate 5-kinase assay is based on the method of Desrivieres S, et al, (1998, J. Biol. Chem., 273(25) 15787-93). Dried lipids are sonicated into 174µl of assay buffer (25 mM HEPES, pH 7.4, at 25°C, 2 mM MgCl, 0.2 mM EDTA, 1 mM EGTA, 5 mM β-glycerophosphate, 1 mM dithiothreitol, and 120 mM NaCl) to give a final concentration in the assay of 100 mM phosphatidylinositol-4-phosphate and 300 mM phosphatidylserine. Purified MSS4 is added to the assay (4µl), and the reactions are started by the addition of 20µl of assay buffer containing 0.04MBq [γ³³P]-ATP and 100µM ATP. Kinase activities are tested at 30°C for 30 min. The reactions are stopped by the addition of 750µl of chloroform: methanol (1:2). The lipids are then extracted by the addition of 200µl of 2.4M HCl and 900µl chloroform. The lower phase is washed twice with 1ml of upper phase (HCl: chloroform: methanol (47:3:48)). The incorporated γ³³P is then measured by transferring 750µl of the lower phase into a scintillation vial, drying under nitrogen, adding 2ml of scintillation cocktail, and counting on a Beckman scintillation counter.

### Example 4: MSS4 as an essential gene product

### 4.1 Construction of the tetracycline-regulatable expression system

*C. albicans* CAI8 was used as the parental strain for all manipulations. The parental strain was constructed to constitutively express a codon-optimised tetracycline transactivator, consisting of TetR fused to the viral VP16 transcriptional activation domain (Gari E, et al, 1997, Yeast, 13:837-848), from the chromosomal enolase promoter (Mason AB, et al, 1993, J. Bacteriol., 175: 2632-2639). One copy of the target gene, *MSS4*, was disrupted in the transactivator expressing strain using the standard *URA-*blaster method (Fonzi WA & Irwin MY, 1993, Genetics, 134:717-728). The promoter region of the other *MSS4* allele was then replaced with the minimal promoter element containing the tetracycline operator sequence *tetO*. Both *in vivo* and *in vitro* this system enables strong induction and tight repression of *MSS4* gene expression in the absence and presence, respectively, of the tetracycline analogue doxycycline.

### 4.2 In vitro validation experiments

The essential nature of MSS4 was determined by assessing the growth and viability of the *C*. *albicans* strain modified to include a tetracycline-regulatable MSS4-expression system as described above (section 4.1). A single fresh colony (grown at 30°C in rich medium in the absence of tetracycline) was used to streak fresh plates containing synthetic complete medium minus uracil (SC-U) (Qbiogene), plus 2% agar, plus or minus 20µg/ml doxycycline as indicated. Growth was scored after 2 days at 30°C (Figure 1). Very few colonies were observed under the conditions where MSS4 expression was repressed (i.e. in the presence of doxycycline).

Growth of the tetracycline-regulatable MSS4-expression system *C. albicans* strain in liquid YEPD medium was also assessed. The inoculum was a 1:100 dilution of an overnight culture adjusted with PBS to an optical density at 600nm =1 and stored at 4°C. Growth, at 25°C, plus or minus 20µg/ml doxycycline, was measured at 30min intervals over a 43h time period or until the growth had noticeably reached a plateau. Growth curves were recorded in 96 well plates in a Wallac plate reader (600nm, 25°C heated stage, 2mm orbital shaking pattern) (Figure 2). Again, significantly reduced growth was observed under the conditions where MSS4 expression was repressed (i.e. in the presence of doxycycline).

### Example 5: MSS4 as an essential gene product: In vivo validation experiment - Murine model of systemic infection with C. albicans conditional mutants

Several reproducible animal models have been described including those of rat vaginal and oral Candidiasis (Calderone RA & Braun PC, 1991, Microbiol. Rev., 55:1-20). However, the most commonly used model is the murine model of hematogenously inoculated, disseminated Candidiasis (Ghannoum MA, et al, 1995, Infect. Immun., 63:4528-4530). In the murine disseminated model, a single dose of organism is inoculated via the tail vein. The end points for this model are survival of animals and tissue counts of *C. albicans* (generally the kidneys).

To further validate the essential nature of the MSS4 gene, the conditional mutants of *C.albicans* strains (as described in Example 4, section 4.1), wherein the MSS4 gene is under the control of a tetracycline repressible promoter, were tested to determine whether they were attenuated in an immunocompetent murine model of infection (Ghannoum *et al*, 1995) as follows:

Initially the organisms were grown in the absence of DOX, since under these conditions they would express the MSS4 gene. These organisms were then used to inoculate two groups of 12 of mice. One group was treated with DOX, in which expression of the MSS4 gene was repressed, and the second group of mice was treated with water (control) wherein the gene continued to be expressed. The mice used were single sex BALB/c mice, Harlan, 4 weeks old and weighing between 19-22 g.

Infective doses of *C. albicans* were injected into the tail vein. The inocula were from saline-washed fresh stationary phase cultures grown in NGY medium [0.1% neopeptone, 0.4% glucose, 0.1% yeast extract] for 18-24 h at 30°C. Yeasts grown in this way have a viable count of 2x10⁷ CFU/ml ± 0.3x10⁷ CFU/ml and can easily be adjusted to the desired concentration with saline. The concentration was checked by spectrophotometry and verified by viable counts. The volume injected was the same across the doses. An infective dose of 1x10⁶ CFU/mouse was used. This infective dose has previously been shown to give a mean survival time of 5-7 days in BALB/c mice.

Animals were fed food and water *ad libitum* throughout the course of experiment. In the DOX-treated group (+DOX), mice were administered with DOX (2 mg/ml) dissolved in 5% sucrose solution as drinking water from 2 days before the inoculation of *C. albicans* cells. The mice are known to drink approximately 5 ml of sucrose solution every day. Under this regimen, the concentrations of DOX in serum, liver, and kidney are maintained at more than 2 mg/ml of serum, 8 mg/g of liver, and 10 mg/g of kidney, respectively (Nakayama, H., et al., 1998, Microbiology 144:2407-2415.) Percent survival was followed over 28 days with daily body weight monitoring. Differences between the effects of *C. albicans* with the MSS4 gene active (-DOX) or repressed (+DOX) *in vivo* were monitored by mouse survival (see Figure 3), kidney burdens of viable fungi, and changes in body weight relative to baseline.

Kidney counts (colony forming unit counts per gram of kidney tissue) for the (+DOX) group were significantly reduced compared to the control group (-DOX) and these mice also maintained their weight throughout the course of the study.

## Claims

1. A method of screening or testing for candidate compounds against *Candida* species, that impair *C. albicans* MSS4 1-phosphatidylinositol-4-phosphate 5-kinase enzyme (MSS4) function, comprising:
a) providing *C. albicans* MSS4;
b) providing one or more candidate compounds;
c) contacting said MSS4 with said one or more candidate compounds; and
d) determining the interaction of the candidate compound with said MSS4.

2. A method according to claim 1 wherein the *C. albicans* MSS4 comprises a fragment, a function-conservative variant, an active fragment or a fusion protein of MSS4.

3. A modified eukaryotic *C. albicans* cell(s) wherein the cell(s) expresses *C. albicans* MSS4 under the control of a heterologous promoter.

4. The cell according to claim 3, wherein the MSS4 is homologous.

5. The cell according to claim 3, wherein the MSS4 comprises a fragment, a function-conservative variant, an active fragment or a fusion protein of MSS4.

6. A method of screening or testing for candidate compounds against *Candida* species, that impair *C. albicans* MSS4 1-phosphatidylinositol-4-phosphate 5-kinase enzyme (MSS4) function, comprising:
a) providing *C. albicans* MSS4 in a eukaryotic cell(s) as defined in claim 3 or 4;
b) providing one or more candidate compounds;
c) contacting said eukaryotic cell(s) with said one or more candidate compounds; and
d) determining the interaction of the candidate compound with said MSS4 by assessing the effect on growth or viability of said cells.

## Patentansprüche

1. Verfahren zum Screenen oder Testen auf Wirkstoffkandidaten gegen die Spezies *Candida*, welche die Funktion des *C. albicans*-MSS4 1-Phosphatidylinositol-4-phosphat-5-kinase-Enzyms (MSS4) beeinträchtigen, umfassend:
a) Bereitstellen von *C. albicans*-MSS4;
b) Bereitstellen von einem oder mehreren Wirkstoffkandidaten;
c) Inkontaktbringen des MSS4 mit dem einen oder den mehreren Wirkstoffkandidaten; und
d) Bestimmen der Wechselwirkung des Wirkstoffkandidaten mit dem MSS4.

2. Verfahren nach Anspruch 1, wobei das *C. albicans*-MSS4 ein Fragment, eine funktionskonservative Variante, ein aktives Fragment oder ein Fusionsprotein von MSS4 umfasst.

3. Modifizierte eukaryontische *C.albicans*-Zelle(n), wobei die Zelle(n) *C.albicans*-MSS4 unter der Steuerung eines heterologen Promoters exprimiert(exprimieren).

4. Zelle nach Anspruch 3, wobei das MSS4 homolog ist.

5. Zelle nach Anspruch 3, wobei das MSS4 ein Fragment, eine funktionskonservative Variante, ein aktives Fragment oder ein Fusionsprotein von MSS4 umfasst.

6. Verfahren zum Screenen oder Testen auf Wirkstoffkandidaten gegen die Spezies *Candida*, welche die Funktion des *C. albicans*-MSS4 1-Phosphatidylinositol-4-phosphat-5-kinase-Enzyms (MSS4) beeinträchtigen, umfassend:
a) Bereitstellen von *C. albicans*-MSS4 in (einer) eukaryontischen Zelle(n), wie in Anspruch 3 oder 4 definiert;
b) Bereitstellen von einem oder mehreren Wirkstoffkandidaten;
c) Inkontaktbringen der eukaryontischen Zelle(n) mit dem einen oder den mehreren Wirkstoffkandidaten; und
d) Bestimmen der Wechselwirkung des Wirkstoffkandidaten mit dem MSS4 durch Beurteilen der Auswirkung auf das Wachstum oder die Lebensfähigkeit dieser Zellen.

## Revendications

1. Procédé de criblage ou de test de composés candidats dirigés contre l'espèce *Candida,* qui détériorent la fonction de l'enzyme 1-phosphatidylinositol-4-phosphate 5-kinase MSS4 de *C. albicans* (MSS4), comprenant les opérations consistant à :
a) se procurer MSS4 de *C. albicans ;*
b) se procurer un ou plusieurs composés candidats ;
c) mettre en contact ladite MSS4 avec ledit ou lesdits composés candidats ; et
d) déterminer l'interaction du composé candidat avec ladite MSS4.

2. Procédé selon la revendication 1, dans lequel la MSS4 de *C. albicans* comprend un fragment, un variant à conservation de fonction, un fragment actif ou une protéine de fusion de MSS4.

3. Cellule ou cellules de *C. albicans* eucaryotes modifiées, la ou les cellules exprimant MSS4 de *C. albicans* sous le contrôle d'un promoteur hétérologue.

4. Cellule selon la revendication 3, dans laquelle la MSS4 est homologue.

5. Cellule selon la revendication 3, dans laquelle la MSS4 comprend un fragment, un variant à conservation de fonction, un fragment actif ou une protéine de fusion de MSS4.

6. Procédé de criblage ou de test de composés candidats dirigés contre l'espèce *Candida*, qui détériorent la fonction de l'enzyme 1-phosphatidylinositol-4-phosphate 5-kinase MSS4 de *C. albicans* (MSS4), comprenant les opérations consistant à :
a) se procurer MSS4 de *C. albicans* dans une ou des cellules eucaryotes, telle(s) que définie(s) à la revendication 3 ou 4 ;
b) se procurer un ou plusieurs composés candidats ;
c) mettre en contact ladite ou lesdites cellules eucaryotes avec ledit ou lesdits composés candidats ; et
d) déterminer l'interaction du composé candidat avec ladite MSS4 par l'évaluation de l'effet sur la croissance ou la viabilité desdites cellules.
